# EUROPÄISCHE PATENTSCHRIFT

(11) **EP 2 510 888 B1**
(45) Veröffentlichungstag und Bekanntmachung des Hinweises auf die Patenterteilung: **17.12.2014**
(21) Anmeldenummer: 12002392.4
(22) Anmeldetag: 02.04.2012
(51) Int. Cl.: A61B 17/29

(54) **Handhabungseinrichtung für ein mikroinvasiv-chirurgisches instrument**
Handling device for a micro-invasive surgical instrument
Dispositif de manipulation pour un instrument de chirurgie micro-invasive

(30) Priorität: 11.04.2011 DE 102011007121
(43) Veröffentlichungstag der Anmeldung: 17.10.2012
(73) Patentinhaber: Karl Storz GmbH & Co. KG, 78532 Tuttlingen (DE)
(72) Erfinder: Kärcher, Daniel, 78315 Radolfzell (DE); Stefan, Jochen, 88639 Wald (DE)

(56) Entgegenhaltungen:
- EP-A1- 1 005 836
- WO-A1-2009/046490
- DE-B3- 10 224 190
- DE-C1- 19 809 120
- US-A1- 2008 046 003

## Beschreibung

Die vorliegende Erfindung bezieht sich auf eine Handhabungseinrichtung für ein mikroinvasiv-chirurgisches Instrument, ein mikroinvasiv-chirurgisches Instrument und dabei insbesondere auf Merkmale zur Kopplung eines Schafts und einer Übertragungsstange mit der Handhabungseinrichtung.

Viele mikroinvasiv-chirurgische Instrumente umfassen einen langen und dünnen Schaft, ein Werkzeug am distalen Ende des Schafts und eine Handhabungseinrichtung am proximalen Ende des Schafts. Das Werkzeug umfasst beispielsweise eine Fass-, Präparier-, Biopsie- oder andere Zange, eine Schere oder einen Nadelhalter mit mindestens zwei geraden oder gekrümmten Backen, Schneiden oder anderen Maulteilen, von denen mindestens eine bzw. eines bewegbar ist. Alternativ umfasst das Werkzeug eine andere Wirkeinrichtung, beispielsweise einen Manipulator mit einem Finger bzw. einer fingerförmigen Einrichtung oder eine Elektrode in Hakenform oder in anderer Gestalt. Der Schaft enthält (mindestens) eine Übertragungsstange, die in der Regel in einem geschlossenen Kanal im Inneren des Schafts angeordnet ist. Die Handhabungseinrichtung umfasst ein oder mehrere relativ zueinander bewegbare Betätigungseinrichtungen, beispielsweise zwei Griffteile, die medizinisches Personal mit einer Hand relativ zueinander bewegen kann. Das proximale Ende und das distale Ende der Übertragungsstange sind so mit der Betätigungseinrichtung bzw. mit dem Werkzeug gekoppelt, dass eine vom medizinischen Personal auf die Betätigungseinrichtungen ausgeübte Kraft oder eine durch medizinisches Personal hervorgerufene relative Bewegung der Betätigungseinrichtungen auf das Werkzeug übertragen werden, beispielsweise um Backen aufeinander zu zu bewegen bzw. zusammenzudrücken.

Bei der Verwendung eines derartigen mikroinvasiv-chirurgischen Instruments werden das Werkzeug und ein Teil des Schafts beispielsweise durch eine natürliche oder künstliche Körperöffnung in einen natürlichen oder künstlichen Hohlraum im Körper eines Patienten eingeführt. Die Entwicklung mikroinvasiver Operationstechniken geht dahin, immer kleinere und vor allem immer weniger Zugänge zu verwenden. Um beispielsweise in der laparoskopischen Chirurgie durch einen einzigen Trokar hindurch mit einem Endoskop und zwei Instrumenten arbeiten zu können, können Instrumente mit gekrümmten Schäften verwendet werden. Ein Instrument mit einem gekrümmten Schaft kann allerdings innerhalb des Zugangs nicht ohne weiteres um seine Längsachse gedreht werden, um die Orientierung des Werkzeugs an seinem distalen Ende zu verändern.

In der DE 10 2006 038 516 A1 ist ein medizinisches Rohrschaftinstrument beschrieben, bei dem ein Werkzeug 5, ein Schaft 3 und eine Handhabe 2 zur Reinigung voneinander getrennt werden können.

In der DE 10 2008 015 418 A1 ist ein medizinisches Instrument mit einem gekrümmten Schaft beschrieben. Ein Maulteil ist mittels eines Bajonettverschlusses mit einem Schaft lösbar verbunden. Im verbundenen Zustand ist das Maulteil gegenüber dem Schaft drehbar. Der Schaft ist lösbar mit einer Handhabe verbunden. Mittels eines Handrads, das drehfest mit einem äußeren Schaftrohr verbunden ist, kann der gekrümmte Schaft gegenüber der Handhabe gedreht werden. Ein Innenrohr ist mit einem weiteren Handrad an der Handhabe verbunden. Das Instrument kann als unipolares oder bipolares HF-Instrument ausgebildet sein.

In der DE 10 2008 052 623 A1 ist ein chirurgisches Instrument mit einer Mauleinheit, einem Schaft und einer Griffeinheit beschrieben. Die Mauleinheit ist lösbar am Ende eines Schaftrohres des Schafts befestigt und gegenüber diesem drehbar.

Werkzeug, Schaft und Handhabungseinrichtung eines mikroinvasiv-chirurgischen Instruments sollen ohne Verwendung von Hilfsmitteln voneinander trennbar und miteinander verbindbar bzw. koppelbar sein, um eine einfache und gründliche Reinigung des Instruments zu ermöglichen. Beispielsweise aus der DE 10 2006 038 516 A1 ist es bekannt, das Werkzeug und das distale Ende des Schafts so auszubilden, dass das Werkzeug in einer über-offenen Montagestellung am Schaft montiert und demontiert werden kann. Insbesondere bei einer Drehbarkeit bzw. Rotierbarkeit des Werkzeugs gegenüber dem Schaft im gekoppelten Zustand sind jedoch einige Aspekte sowohl der Kopplung des Werkzeugs mit dem Schaft als auch der Kopplung des Schafts mit der Handhabungseinrichtung bislang nicht vollständig befriedigend gelöst.

WO 2009/046490 A1 offenbart eine Einrichtung gemäß dem Oberbegriff des Anspruchs 1.

Eine Aufgabe der vorliegenden Erfindung besteht darin, eine verbesserte Handhabungseinrichtung für ein mikroinvasiv-chirurgisches Instrument und ein verbessertes mikroinvasiv-chirurgisches Instrument zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, eine Handhabungseinrichtung mit einer zwischen einer Montageposition und einem vorbestimmten proximalen Bereich von Arbeitspositionen verschiebbaren Stangenkupplung so auszubilden, das ein in einer Schaftkupplung der Handhabungseinrichtung arretiertes proximales Ende eines Schafts mittelbar oder unmittelbar die Stangenkupplung in dem vorbestimmten Bereich von Arbeitspositionen hält. Eine Arretierung des proximalen Endes eines Schafts in der Schaftkupplung der Handhabungseinrichtung bedeutet insbesondere eine Arretierung hinsichtlich einer Bewegung des proximalen Endes des Schafts parallel zu seiner Längsachse. Der Schaft ist insbesondere auch dann um die Längsachse seines proximalen Endes relativ zur Handhabungseinrichtung rotierbar, wenn er an der Handhabungseinrichtung arretiert ist.

Eine Handhabungseinrichtung für ein mikroinvasiv-chirurgisches Instrument umfasst eine Griffeinrichtung zum manuellen Halten und Führen der Handhabungseinrichtung, eine Schaftkupplung zum lösbaren mechanischen Koppeln der Handhabungseinrichtung mit einem proximalen Ende eines Schafts und eine Stangenkupplung, die in der Handhabungseinrichtung zwischen einer vorbestimmten distalen Montageposition und einem vorbestimmten proximalen Bereich von Arbeitspositionen linear verschiebbar ist, zum lösbaren mechanischen Koppeln mit einem proximalen Ende einer Übertragungsstange, die in einem mit der Schaftkupplung gekoppelten Schaft zumindest entweder verschiebbar oder rotierbar ist, wobei die verschiebbare Stangenkupplung ausgebildet ist, um in der vorbestimmten Montageposition ein proximales Ende einer Übertragungstange aufzunehmen oder freizugeben, und um in dem vorbestimmten Arbeitsbereich von Positionen der Stangenkupplung ein in die Stangenkupplung eingesetztes proximales Ende einer Übertragungsstange zu halten, und wobei die Schaftkupplung ausgebildet ist, um ein in die Schaftkupplung eingesetztes proximales Ende eines Schafts in einer vorbestimmten Position zu arretieren, in der das proximale Ende des Schafts die Stangenkupplung in dem vorbestimmten Bereich von Arbeitspositionen hält.

Die Stangekupplung ist entlang eines gekrümmten oder geraden Pfads linear verschiebbar. Der gerade Pfad, entlang dessen die Stangekupplung verschiebbar ist, ist insbesondere parallel zu einer Längsachse eines in die Handhabungseinrichtung eingesetzten oder einzusetzenden proximalen Endes einer Übertragungsstange. Mit der Längsachse eines Schafts, einer Übertragungsstange oder eines proximalen Endes eines Schafts oder einer Übertragungsstange ist insbesondere die Achse gemeint, zu der der betreffende Gegenstand rotationssymmetrisch oder im Wesentlichen rotationssymmetrisch ist. Im Fall eines gekrümmten Schafts oder einer biegeflexiblen Übertragungsstange beziehen sich diese Aussagen insbesondere auf deren Enden, die in der Regel zumindest abschnittsweise gerade bzw. nicht gekrümmt sind.

Die Stangenkupplung ist insbesondere ausgebildet, um im Arbeitsbereich das proximale Ende einer Übertragungsstange formschlüssig und/oder kraftschlüssig zu halten. Die Stangenkupplung ist insbesondere zur Kopplung mit einer biegeflexiblen Übertragungsstange ausgebildet. Der vorbestimmte Arbeitsbereich von Positionen der Stangenkupplung liegt insbesondere proximal der Montageposition. Der vorbestimmte Arbeitsbereich kann ausgedehnt, insbesondere über mehrere Millimeter ausgedehnt sein, Alternativ kann der Arbeitsbereich klein sein oder - im Rahmen der erreichbaren oder verwendeten Präzision - nur eine Position umfassen. Neben der erwähnten Montageposition können weitere Montagepositionen innerhalb eines insbesondere zusammenhängenden Intervalls bzw. Bereichs (Montagebereich) vorgesehen sein.

Die Stangenkupplung kann zusätzlich um eine Längsachse eines mit der Handhabungseinrichtung gekoppelten oder zu koppelnden Schafts rotierbar sein.

Eine Handhabungseinrichtung, wie sie hier beschrieben ist, ist insbesondere zur Verwendung bzw. Kombination mit einer Übertragungsstange zumindest entweder zum Übertragen einer translatorischen Bewegung und einer entsprechenden Schub- oder Zugkraft oder zum Übertragen einer rotatorischen Bewegung und eines entsprechenden Drehmoments zum distalen Ende der Übertragungsstange vorgesehen und ausgebildet.

Das proximale Ende einer Übertragungsstange, für die die Handhabungseinrichtung vorgesehen und ausgebildet ist, kann einen innerhalb eines vorbestimmten Bereichs konstanten Querschnitt aufweisen. Durch einen nicht-rotationssymmetrischen Querschnitt des proximalen Endes der Übertragungsstange kann ein Drehmoment zwischen Orientierungseinrichtung und/oder Stangenkupplung einerseits und dem proximalen Ende der Übertragungsstange andererseits formschlüssig übertragen werden. Eine translatorische Bewegung und eine entsprechende Kraft können kraft- bzw. reibschlüssig zwischen der Stangenkupplung und dem proximalen Ende der Übertragungsstange übertragen werden. Eine Druckkraft bzw. eine Schubkraft in Richtung nach distal kann auch formschlüssig auf das proximale Ende der Übertragungsstange übertragen werden.

Wenn das proximale Ende der Übertragungsstange in dem Bereich, der für eine Anordnung in der Stangenkupplung vorgesehen ist, einen nicht-konstanten Querschnitt aufweist, können eine translatorische Bewegung und eine entsprechende Kraft alternativ oder zusätzlich formschlüssig zwischen Stangenkupplung und proximalem Ende der Übertragungsstange übertragen werden. Insbesondere weist das proximale Ende der Übertragungsstange eine oder mehrere Hinterschneidungen und/oder Ausnehmungen auf, in die die Stangenkupplung eingreifen kann.

Eine Handhabungseinrichtung, wie sie hier beschrieben ist, weist insbesondere ferner einen Schieber auf, der in der Handhabungseinrichtung zwischen einer vorbestimmten distalen Montageposition und einer vorbestimmten proximalen Arbeitsposition verschiebbar ist, wobei der Schieber ausgebildet und angeordnet ist, um von einem in der Schaftkupplung arretierten proximalen Ende eines Schafts in der vorbestimmten proximalen Arbeitsposition gehalten zu werden.

Der Schieber ist insbesondere entlang einer Geraden verschiebbar, die parallel zur Längsachse eines proximalen Endes einer in die Handhabungseinrichtung eingesetzten oder einzusetzenden Übertragungsstange ist. Der Schieber kann zusätzliche Freiheitsgrade bei Entwurf bzw. Konstruktion der Handhabungseinrichtung und eines Schafts und einer Übertragungsstange, die mit der Handhabungseinrichtung verwendbar sein sollen, schaffen. Insbesondere kann der Schieber eine Distanz zwischen dem proximalen Ende eines in die Handhabungseinrichtung eingesetzten Schafts und dem distalen Ende der Stangenkupplung oder einem anderen Ort, an dem der Schieber auf die Stangenkupplung wirkt, überbrücken. Damit kann der Schieber auf einfache und zuverlässige Weise ermöglichen, dass bei in der Handhabungseinrichtung verriegeltem bzw. arretiertem Schaft die Position der Stangenkupplung im Arbeitsbereich gehalten wird, in dem die Stangenkupplung das proximale Ende der Übertragungsstange hält.

Darüber hinaus kann der Schieber, wie nachfolgend und insbesondere anhand der Ausführungsbeispiele deutlich wird, zusätzliche Funktionen aufweisen bzw. übernehmen. Damit kann der Schieber eine besonders kompakte Bauweise der Handhabungseinrichtung ermöglichen.

Der Schieber weist insbesondere eine Durchgangsöffnung auf, die sich von distal nach proximal erstreckt, wobei der Querschnitt der Durchgangsöffnung von distal nach proximal zumindest abschnittsweise stetig variiert.

Der Querschnitt der Durchgangsöffnung verengt sich insbesondere von distal nach proximal. Distal nimmt die Durchgangsöffnung das proximale Ende einer Übertragungsstange, insbesondere in jeder beliebigen Orientierung, auf. Von distal nach proximal variiert der Querschnitt der Durchgangsöffnung so, dass ein proximales Ende einer Übertragungsstange mit einer ursprünglich innerhalb eines vorbestimmten Winkelbereichs beliebigen Orientierung bei einer Bewegung von distal nach proximal in eine vorbestimmte Orientierung rotiert wird. Dazu variiert der Querschnitt der Durchgangsöffnung insbesondere kontinuierlich oder abschnittsweise kontinuierlich.

Die Durchgangsöffnung des Schiebers weist insbesondere eine Gleitfläche auf, die nicht parallel zu der Richtung ist, in der ein Schaft und eine Übertragungsstange in die Handhabungseinrichtung einzuführen sind. Insbesondere sind mehrere Gleitflächen vorgesehen. Jede Gleitfläche kann eben oder schraubenförmig sein, um bei einer Bewegung eines proximalen Endes einer Übertragungsstange von distal nach proximal in der Durchgangsöffnung eine Rotation in eine vorbestimmte Orientierung oder in eine von mehreren vorbestimmten Orientierungen zu bewirken.

Beispielsweise weist der Schieber in der Durchgangsöffnung zwei ebene Gleitflächen auf, wodurch sich die Durchgangsöffnung von distal nach proximal keilförmig verengt. Der Abstand der beiden Gleitflächen ist am distalen Ende der Durchgangsöffnung insbesondere so gewählt, dass das proximale Ende einer Übertragungsstange in jeder beliebigen Orientierung am distalen Ende in die Durchgangsöffnung eingeführt werden kann. Am proximalen Ende weisen die beiden Gleitflächen insbesondere einen Abstand auf, der so gewählt ist, dass ein proximales Ende einer Übertragungsstange nur in zwei sich um 180° unterscheidenden vorbestimmten Orientierungen ganz durch die Durchgangsöffnung hindurchgeführt werden kann.

Die Durchgangsöffnung kann alternativ am distalen Ende so ausgebildet sein, dass das proximale Ende einer Übertragungsstange nur bei einer Orientierung innerhalb von einem oder mehreren Winkelbereichen in die Durchgangsöffnung eingeführt werden kann, wobei ein proximales Ende einer Übertragungsstange mit einer Orientierung innerhalb eines oder mehrerer kleiner, vorbestimmter Totbereiche nicht in die Durchgangsöffnung eingeführt werden kann.

Gleitflächen, insbesondere ebene und keilförmig aufeinander zulaufende Gleitflächen, können einfach herstellbare Mittel zur automatischen Rotation eines proximalen Endes einer Übertragungsstange in eine vorbestimmte Orientierung sein, die bei geringen Herstellungskosten eine zuverlässige Wirkung erzielen.

Bei einer Handhabungseinrichtung, wie sie hier beschrieben ist, ist der Schieber insbesondere relativ zu der Handhabungseinrichtung um eine Achse rotierbar, wobei der Schieber ferner ausgebildet ist, um ein Drehmoment auf eine mit der Stangenkupplung gekoppelten Übertragungsstange zu übertragen.

Insbesondere ist der Schieber um eine Längsachse eines in die Handhabungseinrichtung eingesetzten proximalen Endes eines Schafts bzw. eines proximalen Endes einer Übertragungsstange in dem Schaft rotierbar. Zur Übertragung eines Drehmoments auf eine mit der Stangenkupplung gekoppelte Übertragungsstange ist der Schieber insbesondere an seinem proximalen Ende formschlüssig mit dem proximalen Ende der vorgesehenen Übertragungsstange ausgebildet. Beispielsweise weist das proximale Ende der Übertragungsstange eine Abflachung mit zwei parallelen Oberflächen in einem vorbestimmten Abstand auf, und der Schieber weist an seinem proximalen Ende zwei gegenüberliegende Flächen mit dem vorbestimmten Abstand oder einem geringfügig größeren Abstand auf.

Der Schieber ist insbesondere mittelbar oder unmittelbar mit einem Drehrad oder einer anderen Betätigungseinrichtung an der Handhabungseinrichtung gekoppelt, mittels derer der Schieber und damit eine Übertragungsstange eines in die Handhabungseinrichtung eingesetzten Schafts gedreht bzw. ein Drehmoment auf diese übertragen werden kann.

In den Schieber können somit mehrere Funktionen integriert sein, insbesondere die automatische Rotation eines proximalen Endes einer Übertragungsstange in eine vorbestimmte Orientierung, die Verriegelung der Stangenkupplung und die Übertragung eines Drehmoments auf eine eingesetzte Übertragungsstange. Diese Integration bzw. Verwirklichung mehrerer Funktionen in dem Schieber ermöglicht einen besonders einfachen, kostengünstig realisierbaren, kompakten und robusten Aufbau der Handhabungseinrichtung.

Bei einer Handhabungseinrichtung, wie sie hier beschrieben ist, umfasst die Stangenkupplung insbesondere eine bewegbare Greifbacke zum Halten eines proximalen Endes einer Übertragungsstange, wobei die Stangenkupplung so ausgebildet ist, dass die Greifbacke bei einer vorbestimmten Montageposition der Stangenkupplung ein proximales Ende einer Übertragungsstange aufnehmen oder freigeben und in einem vorbestimmten Arbeitsbereich von Positionen der Stangenkupplung ein proximales Ende einer Übertragungstange halten kann.

Die Stangenkupplung umfasst insbesondere zwei oder mehr symmetrisch angeordnete Greifbacken. Alternativ umfasst die Stangenkupplung nur eine Greifbacke oder eine asymmetrische Anordnung von Greifbacken. Jede Greifbacke ist insbesondere um eine zugeordnete Achse zwischen einer Montageposition und einer Halteposition schwenkbar. In der Montageposition der Greifbacke bzw. in den Montagepositionen der Greifbacken kann ein proximales Ende einer Übertragungsstange in die Stangenkupplung eingesetzt und aus dieser entnommen werden. In der Halteposition der Greifbacke bzw. in den Haltepositionen der Greifbacke kann ein proximales Ende einer Übertragungsstange formschlüssig und/oder kraft- bzw. reibschlüssig von der Greifbacke bzw. den Greifbacken gehalten werden kann.

Insbesondere ist die Greifbacke oder sind die Greifbacken ausgebildet, um eine Klaue bzw. einen Abschnitt mit einem vergrößerten Querschnitt am proximalen Ende einer Übertragungsstange zu umfassen, um eine formschlüssige Verbindung mit dem proximalen Ende der Übertragungsstange zu bilden. Etwas allgemeiner ausgedrückt kann die Greifbacke einen konkaven Bereich aufweisen, in den ein entsprechender konvexer Bereich am proximalen Ende der Übertragungsstange eingreifen kann. Alternativ kann die Greifbacke oder können die Greifbacken ausgebildet sein, um in eine Verjüngung oder einen konkaven Bereich am oder nahe dem proximalen Ende der Übertragungsstange einzugreifen, um eine formschlüssige Verbindung mit dem proximalen Ende der Übertragungsstange zu bilden. Ferner können sowohl die Greifbacke oder die Greifbacken als auch das proximale Ende der Übertragungsstange jeweils einen konvexen Bereich (oder mehrere konvexe Bereiche) und jeweils einen konkaven Bereich (oder mehrere konkave Bereiche) aufweisen, wobei jeweils ein konvexer Bereiche an der Greifbacke ausgebildet ist, um in einen konkaven Bereich am proximalen Ende der Übertragungsstange einzugreifen, und wobei jeweils ein konvexer Bereiche am proximalen Ende der Übertragungsstange ausgebildet ist, um in einen konkaven Bereich an der Greifbacke einzugreifen.

Die Handhabungseinrichtung kann ferner einen Führungsstift und eine Steuernut umfassen, wobei entweder der Führungsstift oder die Steuernut an der Greifbacke angeordnet ist und wobei der Führungsstift in die Steuernut eingreift, um abhängig von der Position der Stangenkupplung die Greifbacke in unterschiedlichen Positionen zu halten.

Insbesondere sind der Führungsstift und die Steuernut so ausgebildet und so angeordnet, dass die Greifbacke in der Montageposition der Stangenkupplung von der vorgesehenen Position eines proximalen Endes einer Übertragungsstange weggeschwenkt und in Positionen im vorbestimmten Arbeitsbereich zum proximalen Ende der Übertragungsstange hin geschwenkt und mit der Übertragungsstange in formschlüssiger Verbindung ist. Insbesondere sind die Steuernut an der Handhabungseinrichtung in Richtung der Verschiebbarkeit der Stangenkupplung unverschiebbar und der Führungsstift an der Greifbacke angeordnet.

Die Steuernut kann mit der gesamten Stangenkupplung und gegebenenfalls mit dem Schieber rotierbar sein, insbesondere um die Längsachse eines in die Stangenkupplung eingesetzten oder einzusetzenden proximalen Endes einer Übertragungsstange. Im Falle mehrerer Greifbacken weist insbesondere jede Greifbacke einen oder mehrere Führungsstifte auf. Die Greifbacke oder jede Greifbacke von mehreren Greifbacken weist insbesondere zwei gegenüberliegende Führungsstifte auf, die in gegenüberliegende und parallel verlaufende Steuernuten eingreifen.

Bei einer Handhabungseinrichtung, bei der die Stangenkupplung eine bewegbare Greifbacke zum Halten eines proximalen Endes einer Übertragungsstange umfasst, und bei der der Schieber zwischen einer vorbestimmten distalen Montageposition und einer vorbestimmten proximalen Arbeitsposition verschiebbar ist, ist insbesondere ein Oberflächenbereich am Schieber ausgebildet, um in der vorbestimmten proximalen Arbeitsposition des Schiebers einen mechanischen Anschlag für die Greifbacke der Stangenkupplung zu bilden, der den vorbestimmten Arbeitsbereich begrenzt.

Insbesondere ist eine proximale Stirnfläche des Schiebers als Anschlag ausgebildet. Alternativ kann ein anderer Bereich der Oberfläche des Schiebers, der teilweise oder vollständige nach proximal orientiert ist, als Anschlag für einen proximal des Bereichs angeordneten Teils der Stangenkupplung ausgebildet sein.

Die Handhabungseinrichtung ist also insbesondere so ausgebildet, dass die Greifbacke der Stangenkupplung an der als Anschlag ausgebildeten Oberflächenbereich des Schiebers anliegt, wenn der Schieber sich in seiner Arbeitsposition und die Stangekupplung sich in der distalsten Position innerhalb des vorbestimmten Arbeitsbereichs befinden. Insbesondere wenn die Greifbacke oder die Greifbacken am distalen Ende der Stangenkupplung angeordnet sind, kann der mechanische Anschlag der Greifbacken der Stangenkupplung am Schieber eine konstruktiv einfache und zuverlässige Lösung darstellen.

Ein mikroinvasiv-chirurgisches Instrument umfasst eine Handhabungseinrichtung, wie sie hier beschrieben ist, einen Schaft mit einem proximalen Ende, das zur lösbaren Kopplung mit der Handhabungseinrichtung ausgebildet ist, und einem distalen Ende, das mit einem Werkzeug mechanisch verbunden oder verbindbar ist, und eine Übertragungsstange zum Übertragen zumindest entweder einer Kraft oder eines Drehmoments von der Handhabungseinrichtung zum distalen Ende des Schafts.

Der Schaft des mikroinvasiv-chirurgischen Instruments kann gerade oder gekrümmt, starr oder flexibel sein. Im Fall eines gekrümmten oder flexiblen Schafts ist die Übertragungsstange insbesondere abschnittsweise biegeflexibel. Das Werkzeug umfasst insbesondere eine Fass-, Präparier-, Biopsie- oder andere Zange, eine Schere oder einen Nadelhalter mit mindestens zwei geraden oder gekrümmten Backen, Schneiden oder anderen Maulteilen, von denen mindestens eine bzw. eines bewegbar ist. Alternativ umfasst das Werkzeug eine andere Wirkeinrichtung, beispielsweise einen Manipulator mit einem Finger bzw. einer fingerförmigen Einrichtung oder eine Elektrode in Hakenform oder in anderer Gestalt. Die Übertragungsstange ist insbesondere zum Übertragen einer Zug- und/oder Schubkraft zum Werkzeug am distalen Ende des Schafts ausgebildet. Alternativ oder zusätzlich ist die Übertragungsstange zur Übertragung eines Drehmoments und einer Drehbewegung zu dem Werkzeug ausgebildet. Das proximale Ende der Übertragungsstange weist insbesondere eine zu einer Durchgangsöffnung oder zu einem proximalen Ende einer Durchgangsöffnung in der Orientierungseinrichtung korrespondierende Gestalt auf.

Das proximale Ende des Schafts des mikroinvasiv-chirurgischen Instruments ist insbesondere ausgebildet, um in der vorbestimmten Position mittelbar oder unmittelbar die Stangenkupplung in dem vorbestimmten Arbeitsbereich zu halten.

Mit den hier beschriebenen Merkmalen und Eigenschaften der Handhabungseinrichtung und entsprechenden Merkmalen und Eigenschaften des Schafts und der Übertragungsstange sind eine einfache, schnelle und zuverlässige Montage und effiziente Verwendung des mikroinvasiv-chirurgischen Instruments möglich.

Bei dem mikroinvasiv-chirurgischen Instrument kann die Übertragungsstange ferner ausgebildet sein, um ein Drehmoment von der Handhabungseinrichtung zum distalen Ende des Schafts zu übertragen.

Mittels eines von der Übertragungsstage von der Handhabungseinrichtung zum distalen Ende des Schafts übertragenen Drehmoments kann insbesondere das Werkzeug am dista-len Ende des Schafts um seine Längsachse rotiert bzw. gedreht werden. Damit ist eine Rotation des Werkzeugs unabhängig vom Schaft möglich. Beispielsweise bei einem mikroinvasiven Eingriff mit mehreren Instrumenten mit gekrümmten Schäften in einem Trokar kann jedes einzelne Werkzeug unabhängig von der Orientierung des gekrümmten Schafts, an dessen Ende das Werkzeug angeordnet ist, gedreht werden.

Das proximale Ende des Schafts ist insbesondere ausgebildet, um in der vorbestimmten arretierbaren Position mittelbar oder unmittelbar die Stangenkupplung in dem vorbestimmten Arbeitsbereich zu halten.

Bei dem mikroinvasiv-chirurgischen Instrument ist die Übertragungsstange insbesondere ferner ausgebildet, um ein Drehmoment von der Handhabungseinrichtung zum distalen Ende des Schafts zu übertragen.

Insbesondere ist die Übertragungsstange zur Übertragung eines Drehmoments von der Handhabungseinrichtung zu einem Werkzeug am distalen Ende des Schafts ausgebildet. Die Übertragungsstange ist somit insbesondere hinsichtlich Druck- und Zugbelastungen in Längsrichtung und hinsichtlich Torsion steif bzw. unelastisch. Gleichzeitig kann die Übertragungsstange biegeflexibel sein, insbesondere im Fall eines zumindest abschnittsweise gekrümmten Schafts.

Bei einem mikroinvasiv-chirurgischen Instrument, wie es hier beschrieben ist, können das Werkzeug und das distale Ende des Schafts lösbar miteinander mechanisch koppelbar sein, wobei Werkzeug, Schaft und Übertragungsstange so ausgebildet sind, dass das Werkzeug am distalen Ende des Schafts verriegelt ist, wenn die Position der mit dem proximalen Ende der Übertragungsstange gekoppelten Stangenkupplung in dem vorbestimmten Arbeitsbereich liegt.

Die lösbare Verbindung zwischen Werkzeug und Schaft kann eine Reinigung des Instruments verbessern oder überhaupt erst ermöglichen. Die Verriegelung des Werkzeugs am distalen Ende des Schafts, wenn die Position der mit dem proximalen Ende der Übertragungsstange gekoppelten Stangenkupplung in dem vorbestimmten Arbeitsbereich liegt, kann eine vollständige Zerlegung des Instruments allein aufgrund eines Lösens der Arretierung des Schafts an der Handhabungseinrichtung ermöglichen. Dies kann die Handhabung des Instruments, insbesondere sein Zerlegen und Zusammensetzen deutlich vereinfachen oder verbessern.

Ein mikroinvasiv-chirurgisches Instrument, wie es hier beschrieben ist, umfasst insbesondere ein Werkzeug am distalen Ende des Schafts, wobei das Werkzeug ein gekrümmtes Maulteil umfasst.

Insbesondere umfasst das Werkzeug zwei oder mehr gekrümmte Maulteile. Sofern ein gekrümmtes Maulteil um eine Schwenkachse schwenkbar ist, ist es insbesondere in einer Ebene senkrecht zu der Schwenkachse oder in einer Ebene parallel zur Schwenkachse oder in beiden Richtungen gekrümmt.

Bogenförmig oder sogar schraubenförmig gekrümmte Backen, Schneiden oder andere Maulteile von mikroinvasiv-chirurgischen Instrumenten sind für einige Anwendungen besonders geeignet. Bei der Verwendung eines Werkzeugs mit zwei gekrümmten Maulteilen kann aber im Gegensatz zu einem Werkzeug mit zwei gleichen oder ähnlichen, geraden oder im Wesentlichen geraden Maulteilen eine Rotation um mehr als 90 Grad (bis zu 180 Grad) erforderlich sein, um das Werkzeug relativ zu einem Gegenstand richtig auszurichten. Eine Rotierbarkeit eines Werkzeugs mit einem oder mehreren gekrümmten Maulteilen um eine Längsachse eines Schafts eines Werkzeugs kann deshalb besonders vorteilhaft sein.

### Kurzbeschreibung der Figuren

Nachfolgend werden Ausführungsformen anhand der beigefügten Figuren näher erläutert. Es zeigen:
- Figur 1: eine schematische Darstellung eines mikroinvasiv-chirurgischen Instruments;
- Figur 2: eine schematische Darstellung des mikroinvasiv-chirurgischen Instruments aus Figur 1 in zerlegter Form;
- Figur 3: eine schematische Darstellung einer Handhabungseinrichtung;
- Figur 4: eine schematische Darstellung einer Orientierungseinrichtung;
- Figur 5: eine schematische Darstellung einer weiteren Orientierungseinrichtung;
- Figur 6: eine weitere schematische Darstellung der Handhabungseinrichtung aus Figur 3;
- Figur 7: eine weitere schematische Darstellung der Handhabungseinrichtung aus Figur 3;
- Figur 8: eine weitere schematische Darstellung der Handhabungseinrichtung aus Figur 3;
- Figur 9: eine schematische Darstellung einer weiteren Handhabungseinrichtung;
- Figur 10: eine weitere schematische Darstellung der Handhabungseinrichtung aus Figur 9;
- Figur 11: eine weitere schematische Darstellung der Handhabungseinrichtung aus den Figuren 9 und 10.

### Beschreibung der Ausführungsformen

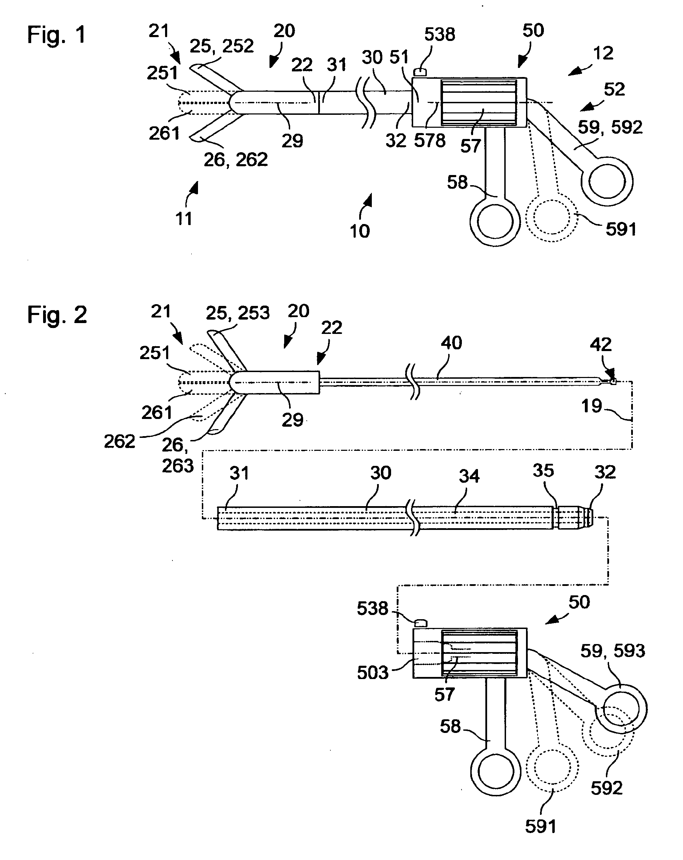

Figur 1 zeigt eine schematische Darstellung eines mikroinvasiv-chirurgischen Instruments 10 mit einem distalen Ende 11 und einem proximalen Ende 12. Das mikroinvasiv-chirurgische Instrument 10 umfasst ein Werkzeug 20, einen Schaft 30 und eine Handhabungseinrichtung 50. Am distalen Ende 21 weist das Werkzeug 20 ein erstes bewegbares Maulteil 25 und ein zweites bewegbares Maulteil 26 auf. Die Maulteile 25, 26 sind in Figur 1 in durchgezogenen Linien in offenen Positionen 252, 262 und in gestrichelten Linien in geschlossenen Positionen 251, 261 dargestellt. Die Maulteile 25, 26 können jeweils gerade oder im Wesentlichen gerade oder in Richtung senkrecht zur Zeichenebene der Figur 1 und/oder - abweichend von der Darstellung in Figur 1 - in der Zeichenebene der Figur 1 gekrümmt sein.

Das proximale Ende 22 des Werkzeugs 20 ist lösbar mechanisch gekoppelt mit einem distalen Ende 31 des Schafts 30. Der Schaft 30 ist in Figur 1 stark verkürzt und der Einfachheit halber gerade dargestellt. Abweichend von der Darstellung in Figur 1 kann der Schaft 30 eben oder räumlich gekrümmt sein. Mit einer innerhalb einer Ebene oder - für einige Anwendungen noch vorteilhafter - räumlich gekrümmten Gestalt des Schafts 30 kann das mikroinvasiv-chirurgische Instrument 10 besonders für mikroinvasiv-chirurgische Eingriffe geeignet sein, bei denen ein Endoskop und ein oder mehrere Instrumente gleichzeitig durch einen einzigen Zugang in eine Körperhöhle eingeführt werden.

Das proximale Ende 32 des Schafts 30 ist mit dem distalen Ende 51 der Handhabungseinrichtung 50 lösbar mechanisch gekoppelt. Zur Handhabung des mikroinvasiv-chirurgischen Instruments 10 weist die Handhabungseinrichtung 50 ein Drehrad 57, ein erstes Griffteil 58 und ein zweites Griffteil 59 auf. Das Drehrad 57 ist zur Steuerung einer Rotation des Werkzeugs 20, insbesondere der Maulteile 25, 26, um eine Längsachse 29 vorgesehen. Bei dem in Figur 1 dargestellten Beispiel ist das Drehrad 57 um eine Achse 578 drehbar, die gleichzeitig die Längsachse des Schafts 30 an seinem proximalen Ende 32 ist. Alternativ kann die Achse 578 parallel zur Längsachse des Schafts 30 an seinem proximalen Ende 32 sein. Ferner weist das Drehrad 57 eine Oberflächenstruktur auf, die auch mit Handschuhen eine zuverlässige Bedienung bzw. Betätigung ermöglicht, beispielsweise die angedeuteten Stege in axialer Richtung. Die Griffteile 58, 59 sind insbesondere - abweichend von der in Figur 1 dargestellten stark stilisierten Gestalt - so angeordnet und geformt, dass medizinisches Personal mit einer Hand beide Griffteile 58, 59 ermüdungsarm greifen und relativ zueinander bewegen kann.

Zumindest eines der beiden Griffteile 58, 59 ist relativ zu den anderen Bestandteilen der Handhabungseinrichtung 50 bewegbar. Bei dem in Figur 1 dargestellten Beispiel sind das erste Griffteil 58 starr und das zweite Griffteil 59 bewegbar angeordnet. Das zweite Griffteil 59 ist insbesondere zwischen einer ersten, in Figur 1 in gestrichelter Linie dargestellten Arbeitsposition 591 und einer zweiten, in Figur 1 in durchgezogener Linie dargestellten Arbeitsposition 592 bewegbar. Das zweite Griffteil 59 der Handhabungseinrichtung 50 ist derart mit den Maulteilen 25, 26 des Werkzeugs 20 mechanisch gekoppelt, dass die Maulteile 25, 26 sich in ihren geschlossenen Positionen 251, 261 befinden, wenn das zweite Griffteil 59 seine erste Arbeitsposition 591 einnimmt, und dass die Maulteile 25, 26 sich in ihren offenen Positionen 252, 262 befinden, wenn das zweite Griffteil 59 seine zweite Arbeitsposition 592 einnimmt.

Figur 2 zeigt eine schematische Darstellung von Bestandteilen bzw. Komponenten des oben anhand der Figur 1 dargestellten mikroinvasiv-chirurgischen Instruments 10, die ohne Verwendung von Werkzeug montiert bzw. zum Instrument zusammengesetzt werden können. Ebenso kann das mikroinvasiv-chirurgische Instrument 10 ohne Werkzeug in die in Figur 2 separat dargestellten Bestandteile bzw. Komponenten zerlegt werden. Durch die die gesamte Figur 2 durchlaufende strichpunktierte Linie 19 ist angedeutet, wie diese Bestandteile bzw. Komponenten zusammenzusetzen sind.

Das Werkzeug 20 ist insbesondere dauerhaft mit einer Übertragungsstange 40 verbunden, die zum Übertragen einer Kraft und eines Drehmoments von der Handhabungseinrichtung 50 zum Werkzeug 20 vorgesehen ist. Das in Figur 2 nicht dargestellte distale Ende der Übertragungsstange 40 ist derart mit den Maulteilen 25, 26 gekoppelt, dass eine Bewegung der Übertragungsstange 40 parallel zur Längsachse 29 des Werkzeugs 20 eine synchrone Bewegung der Maulteile 25, 26 bewirkt.

Am proximalen Ende 22 des Werkzeugs 20 und am distalen Ende 31 des Schafts 30 sind in Figur 2 nicht dargestellte Bajonettkupplungseinrichtungen sowie eine mit der Übertragungsstange 40 gekoppelte Verriegelungseinrichtung vorgesehen. Die Maulteile 25, 26 sind in Figur 2 in durchgezogenen Linien in überoffenen Positionen 253, 263 und in gestrichelten Linien in den bereits oben anhand der Figur 1 beschriebenen geschlossenen und offenen Positionen 251, 252, 261, 262 dargestellt. Wenn die Maulteile 25, 26 sich in den überoffenen Positionen 253, 263 befinden, ist die mit den Maulteilen 25, 26 und dem distalen Ende der Übertragungsstange 40 gekoppelte und in Figur 2 nicht dargestellte Verriegelungseinrichtung inaktiv. In diesem Zustand können die Übertragungsstange 40 in einen für die Übertragungsstange 40 vorgesehenen Kanal 34 im Schaft 30 eingeführt und das proximale Ende 22 des Werkzeugs und das distale Ende 31 des Schafts über die in Figur 2 nicht dargestellten Bajonettkupplungseinrichtungen miteinander lösbar mechanisch verbunden bzw. gekoppelt werden. Ferner kann in diesem entriegelten Zustand eine mechanische Kopplung des proximalen Endes 22 des Werkzeugs 20 und des distalen Endes 31 des Schafts 30 über die in Figur 2 nicht dargestellten Bajonettkupplungseinrichtungen gelöst werden.

Wenn die Maulteile 25, 26 sich in den geschlossenen Positionen 251, 261, in den offenen Positionen 252, 262 oder in dazwischen liegenden Positionen befinden, befindet sich die mit dem distalen Ende der Übertragungsstange 40 und mittelbar mit den Maulteilen 25, 26 gekoppelte Verriegelungseinrichtung in einer Arbeitsposition bzw. in einer Position innerhalb eines Arbeitsbereichs. In der Arbeitsposition bzw. in den Positionen innerhalb des Arbeitsbereichs ist die mechanische Kopplung des proximalen Endes 22 des Werkzeugs 20 mit dem distalen Ende 31 des Schafts 30 über die in Figur 2 nicht dargestellten Bajonettkupplungseinrichtungen verriegelt. Wenn die mechanische Verbindung bzw. Kopplung von Werkzeug 20 und Schaft 30 verriegelt ist, können das Werkzeug 20 und der Schaft 30 nicht oder nicht ohne Weiteres zerstörungsfrei voneinander getrennt werden.

Anstelle der Bajonettkupplungseinrichtungen können das proximale Ende 22 des Werkzeugs 20 und das distale Ende 31 des Schafts 30 andere Kupplungseinrichtungen aufweisen. Auch in diesem Fall ist eine Verriegelungseinrichtung am Werkzeug 20 vorgesehen, die die mechanische Verbindung von Werkzeug 20 und Schaft 30 verriegelt, wenn die Maulteile 25, 26 sich in den überoffenen Positionen 253, 263 befinden.

Wenn die Übertragungsstange 40 in den Kanal 34 des Schafts 30 eingesetzt und das proximale Ende 22 des Werkzeugs 20 mit dem distalen Ende 31 des Schafts 30 mechanisch verbunden bzw. gekoppelt ist, kann das proximale Ende 32 des Schafts 30 mit dem gegenüber dem proximalen Ende 32 des Schafts 30 überstehenden proximalen Ende 42 der Übertragungsstange 40 in die Handhabungseinrichtung 50 eingesetzt werden. Dazu weist die Handhabungseinrichtung 50 eine in Figur 2 durch eine punktierte Linie angedeutete Ausnehmung 503 auf.

Zum Einsetzen des proximalen Endes 32 des Schafts 30 und des proximalen Endes 42 der Übertragungsstange 40 in die Handhabungseinrichtung 50 wird das zweite Griffteil 59 zunächst in eine in Figur 2 in durchgezogener Linie dargestellte Koppelposition 593 gebracht. Wenn sich das zweite Griffteil 59 in der Koppelposition 593 befindet, befindet sich eine in Figur 2 nicht dargestellte Stangenkupplung im Inneren der Handhabungseinrichtung 50 in einer Koppelposition, in der sie das proximale Ende 42 der Übertragungsstange 40 aufnehmen oder freigeben kann. Wenn das proximale Ende 42 der Übertragungsstange 40 ganz in die Handhabungseinrichtung 50 eingeführt sind, wird die in Figur 2 nicht dargestellte Stangenkupplung im Inneren der Handhabungseinrichtung 50 mit dem proximalen Ende 42 der Übertragungsstange 40 mechanisch verbunden bzw. gekoppelt. Dabei geht das zweite Griffteil 59 abhängig von den Positionen der Maulteile 25, 26 (geschlossene Positionen 251, 261, offene Positionen 252, 262 oder dazwischen) in die erste Arbeitsposition 591, die zweite Arbeitsposition 592 oder eine Position zwischen der ersten Arbeitsposition 591 und der zweiten Arbeitsposition 592 über.

Wenn das proximale Ende 32 des Schafts 30 ganz in die Handhabungseinrichtung 50 eingeführt ist, greift ein in Figur 2 nicht dargestellter Riegel in eine umlaufende Nut 35 nahe dem proximalen Ende 32 des Schafts 30 ein und verriegelt damit das proximale Ende 32 des Schafts 30 in einer vorgesehenen Position in der Handhabungseinrichtung 50. Durch die Verriegelung des proximalen Endes 32 des Schafts 30 in der Handhabungseinrichtung 50 wird mittelbar auch die mechanische Kopplung des proximalen Endes 42 der Übertragungsstange 40 mit der in Figur 2 nicht dargestellten Stangenkupplung im Innern der Handhabungseinrichtung 50 verriegelt.

Nach Verriegelung des proximalen Endes 32 des Schafts 30 in der Handhabungseinrichtung 50 und mittelbar des proximalen Endes 42 der Übertragungsstange 40 in der in Figur 2 nicht dargestellten Stangenkupplung in der Handhabungseinrichtung 50 ist das mikroinvasiv-chirurgische Instrument 10 wie in Figur 1 dargestellt konfiguriert. Durch Bewegung des zweiten Griffteils 59 relativ zum ersten Griffteil 58 zwischen den beiden Arbeitspositionen 591, 592 können die Maulteile 25, 26 zwischen den geschlossenen Positionen 251, 261 und den offenen Positionen 252, 262 bewegt werden. Durch Rotation des Drehrads 57 um die Achse 578 können die Maulteile 25, 26, um die Längsachse 29 des Werkzeugs 20 gedreht werden.

Abweichend von den Darstellungen in den Figuren 1 und 2 kann der Schaft 30 nahe seinem proximalen Ende 32 ein weiteres Drehrad aufweisen, das nahe dem distalen Ende 51 der Handhabungseinrichtung 50 angeordnet ist, wenn das proximale Ende 32 des Schafts 30 in die Handhabungseinrichtung 50 eingesetzt ist. Mittels dieses in den Figuren 1 und 2 nicht dargestellten Drehrads kann der Schaft 30 um die Längsachse des proximalen Endes 20 des Schafts 30 gedreht werden. Dies ist insbesondere von Bedeutung, wenn der Schaft 30 abweichend von den Darstellungen in den Figuren 1 und 2 gekrümmt ist. In diesem Fall können der gekrümmte Schaft 30 und das Werkzeug 20 am distalen Ende 31 des gekrümmten Schafts 30 unabhängig voneinander gedreht werden.

Durch Druck auf den Entriegelungsknopf 538 kann der in Figur 2 nicht dargestellte Riegel gegen die Kraft einer Feder verschoben und aus der Nut 35 am Schaft 30 ausgerückt werden. Danach kann das proximale Ende 32 des Schafts 30 aus der Handhabungseinrichtung 50 entnommen werden. Dabei wird auch die Verriegelung des proximalen Endes 42 der Übertragungsstange 40 an der in den Figuren 1 und 2 nicht dargestellten Stangenkupplung in der Handhabungseinrichtung 50 gelöst.

Anstelle eines oder - wie in den Figuren 1 und 2 dargestellt - zweier bewegbarer Maulteile 25, 26 kann das Werkzeug 20 eine andere Wirkeinrichtung aufweisen, insbesondere einen Manipulator, beispielsweise einen fingerförmigen Manipulator, oder eine Elektrode, beispielsweise eine hakenförmige Elektrode.

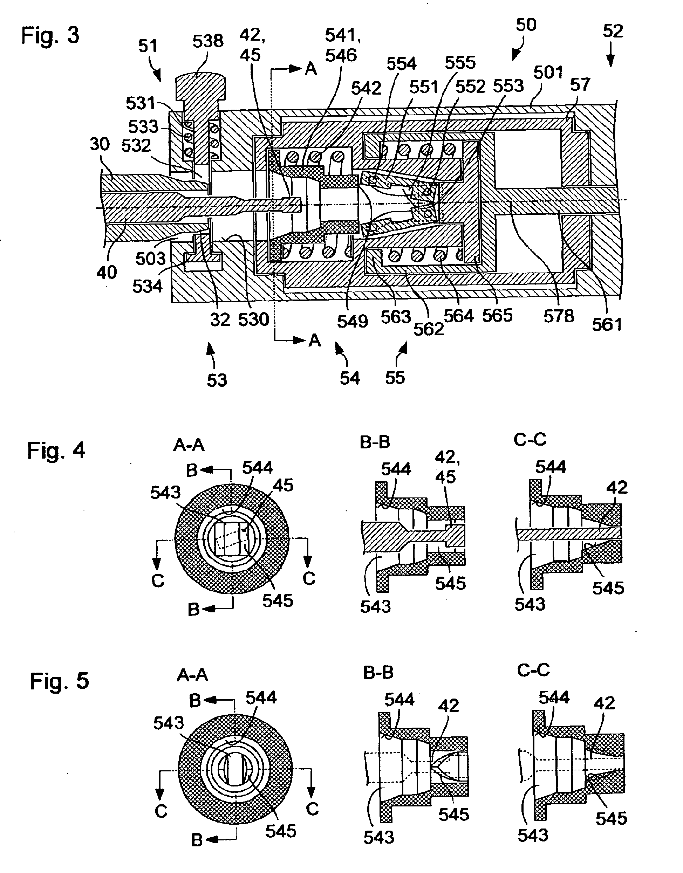

Figur 3 zeigt eine schematische Darstellung eines Schnitts durch ein Ausführungsbeispiel der Handhabungseinrichtung 50 des oben anhand der Figuren 1 und 2 dargestellten mikroinvasiv-chirurgischen Instruments. Die dargestellte Schnittebene ist parallel zu den Zeichenebenen der Figuren 1 und 2 und enthält die bereits in den Figuren 1 und 2 angedeutete Achse 578. Die in den Figuren 1 und 2 dargestellten Griffteile 58, 59 sind in Figur 3 nicht gezeigt.

Die Handhabungseinrichtung 50 umfasst ein Gehäuse 501 mit der vom distalen Ende 51 der Handhabungseinrichtung 50 ausgehenden Ausnehmung 503, die bereits in Figur 2 angedeutet ist. Die Oberfläche der Ausnehmung 503 ist insbesondere im Wesentlichen als zur Achse 578 symmetrische kreiszylindrische Führungsfläche 530 ausgebildet. Ein proximales Ende 32 eines Schafts 30 und ein proximales Ende 42 einer im Schaft 30 angeordneten Übertragungsstange 40 sind in Figur 3 teilweise in die Ausnehmung 503 eingeführt dargestellt.

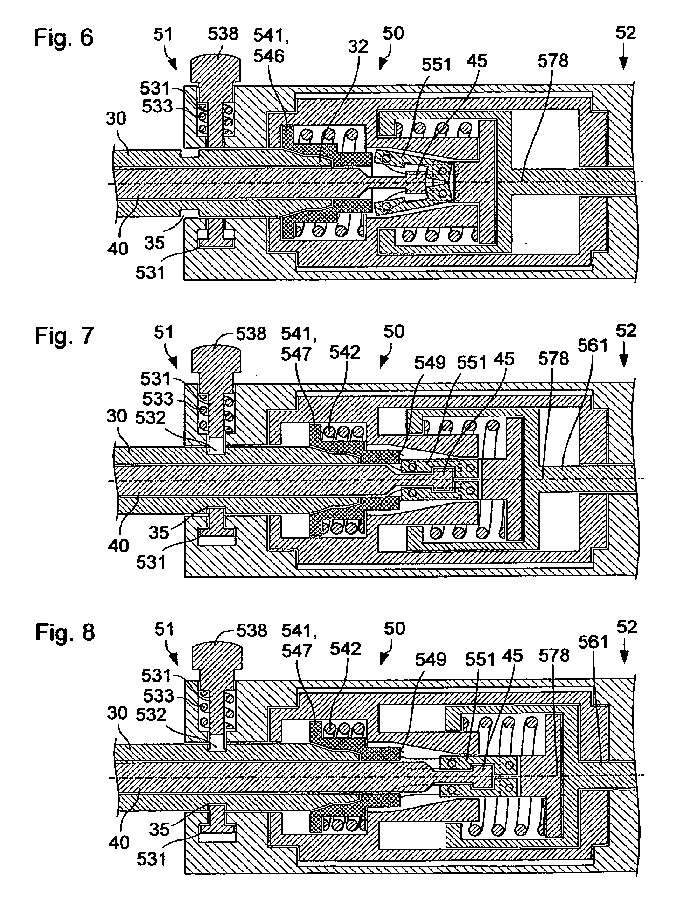

Die vollständige Einführung des proximalen Endes 32 des Schafts 30 und des proximalen Endes 42 der Übertragungsstange 40 in die Handhabungseinrichtung 50, die Verriegelung des Schafts 30 an der Handhabungseinrichtung 50 und die mechanische Kopplung des proximalen Endes 42 der Übertragungsstange 40 mit Elementen der Handhabungseinrichtung 50 sind unten mit Bezug auf die Figuren 6 bis 8 dargestellt. Mit Bezug auf Figur 3 werden zunächst nur die Bestandteile bzw. Elemente der Handhabungseinrichtung 50 beschrieben.

Einige Bestandteile bzw. Elemente der Handhabungseinrichtung 50, die in den Figuren 3 und 6 bis 8 jeweils einstückig dargestellt sind, weil sie in der Handhabungseinrichtung 50 jeweils für sich starre mechanische Einheiten bilden, können abweichend von den Darstellungen - beispielsweise aus fertigungstechnischen Gründen - jeweils aus mehreren formschlüssig, kraftschlüssig oder stoffschlüssig gefügten Elementen zusammengesetzt sein.

Nahe dem distalen Ende 51 umfasst die Handhabungseinrichtung 50 eine Schaftkupplung 53. Die Schaftkupplung 53 wird durch die Führungsfläche 530 und eine Verriegelungseinrichtung zum Verriegeln bzw. Arretieren des Schafts 30 in der Handhabungseinrichtung 50 gebildet. Die Verriegelungseinrichtung umfasst einen Riegel 531, der sich im Wesentlichen quer zur Achse 578 erstreckt und quer zur Achse 578 innerhalb eines vorbestimmten Bereichs linear verschiebbar ist.

An einem Ende weist der Riegel 531 einen Kragen 534 in einem Hohlraum im Gehäuse 501 der Handhabungseinrichtung 50 auf, wobei die Gestalt des Kragens 534 und die Gestalt des Hohlraums die lineare Verschiebbarkeit des Riegels 531 begrenzen. Am anderen Ende weist der Riegel 531 einen Entriegelungsknopf 538 auf, der gegenüber der äußeren Kontur des Gehäuses 501 der Handhabungseinrichtung 50 übersteht. Der Riegel 531 weist eine Öffnung 532 auf, die an die äußere Kontur des Querschnitts des Schafts 30 angepasst ist. Eine Feder 533 zwischen einem Absatz am Gehäuse 501 der Handhabungseinrichtung 50 und einem Absatz am Riegel 531 schiebt den Riegel 531 in die in Figur 3 dargestellte Position.

Durch Druck auf den Entriegelungsknopf 538 oder durch Einwirkung eines konischen Abschnitts am Schaft 30 auf den Rand der Öffnung 532 im Riegel 531 kann der Riegel 531 gegen die Kraft der Feder 533 bis zu einer Position linear verschoben werden, in der der Rand der Öffnung 532 im Riegel 531 nicht mehr in die Ausnehmung 503 hineinragt. In dieser Position des Riegels 531 kann der Schaft 30 in die Ausnehmung 503 eingesetzt oder aus dieser entnommen werden.

Die Handhabungseinrichtung 50 umfasst ferner eine Steuer- und Orientierungseinrichtung 54, die nachfolgend als Orientierungseinrichtung bezeichnet wird, und eine Stangenkupplung 55 innerhalb des im Wesentlichen trommelförmigen Drehrads 57. Die Orientierungseinrichtung 54 und die Stangenkupplung 55 sind ausgebildet, um zusammen mit dem Drehrad 57 um die Achse 578 gedreht zu werden. Die Orientierungseinrichtung 54 und die Stangenkupplung 55 sind also hinsichtlich Rotation um die Achse 578 starr mit dem Drehrad 57 verbunden. Sowohl die Orientierungseinrichtung 54 als auch die Stangenkupplung 55 sind aber innerhalb der Handhabungseinrichtung und relativ zum Drehrad 57 in axialer Richtung bzw. parallel zur Achse 578 jeweils innerhalb eines vorbestimmten Bereichs verschiebbar.

Die Orientierungseinrichtung 54 umfasst einen Schieber 541, der in Figur 3 in einer distalen Montageposition 546 dargestellt ist. Zwischen einem Kragen des Schiebers 541 und dem Drehrad 57 ist eine Feder 542 angeordnet, die den Schieber 541 elastisch in der Montageposition 546 hält. Der Schieber 541 und das Drehrad 57 weisen insbesondere am proximalen Ende des Schiebers 541 in der Umgebung einer proximalen Stirnfläche 549 des Schiebers 541 Flächen auf, die aneinander anliegen und nicht rotationssymmetrisch zur Achse 578 sind. Dadurch wird der Schieber 541 unabhängig von seiner Position in axialer Richtung hinsichtlich der Rotation um die Achse 578 in einer vorbestimmten Orientierung relativ zum Drehrad 57 gehalten.

Die Stangenkupplung 55 umfasst zwei symmetrisch zur Achse 578 angeordnete Greifbacken 551, die über Gelenke 552 an einem Kraftübertragungsbauteil 565 gelenkig befestigt sind. Jede Greifbacke 551 weist eine Ausnehmung 553 mit einer Form, die zur Gestalt einer Klaue 45 am proximalen Ende 42 der Übertragungsstange 40 korrespondiert, auf.

Ferner weist jede Greifbacke 551 einen Führungsstift 554 auf, dessen Achse senkrecht zur Schnittebene der Figur 3 ist. Jedes Ende von jedem der Führungsstifte 554 greift jeweils in eine zugeordnete Steuernut 555 im Drehrad 57 ein. Außer den beiden in Figur 3 dargestellten Steuernuten 555 hinter der Schnittebene der Figur 3 sind zwei in Figur 3 nicht dargestellte Steuernuten am Drehrad 57 vorgesehen, die bezüglich der Schnittebene der Figur 3 zu den in Figur 3 dargestellten Steuernuten 57 spiegelsymmetrisch sind. Die Steuernuten 555 sind zumindest abschnittsweise gekrümmt. Bei einer linearen Verschiebung der Gelenke 552 der Greifbacken 551 parallel zur Achse 578 bewirken die Steuernuten 555 Schwenkbewegungen der Greifbacken 551, die unten mit Bezug auf die Figuren 6 bis 8 beschrieben werden.

Im Inneren des Drehrads 57 sind ferner ein Stab 561 und ein becherförmiges Bauteil 562 angeordnet. Der Stab 561 und das becherförmige Bauteil 562 sind starr miteinander verbunden und parallel zur Achse 578 innerhalb des Drehrads 57 verschiebbar. Zwischen einem radial nach innen ragenden Kragen 563 des becherförmigen Bauteils 562 und einem radial nach außen ragenden Kragen des Kraftübertragungsbauteils 565 ist eine Druckfeder 564 angeordnet. Die Druckfeder 564 begrenzt eine Zugkraft, die von dem Stab 561 über das becherförmige Bauteil 562, die Druckfeder 564, das Kraftübertragungsbauteil 565 und die Greifbacken 551 auf ein mit den Greifbacken 551 gekoppeltes proximales Ende 42 einer Übertragungsstange 40 übertragen werden kann.

Das in Figur 3 nicht dargestellte proximale Ende des Stabs 561 ist - beispielsweise mittels eines Pleuels - so mit dem oben anhand der Figuren 1 und 2 dargestellten zweiten Griffteil 59 gekoppelt, dass eine Schwenkbewegung des zweiten Griffteils 59 eine lineare Bewegung des Stabs 561 und der Stangenkupplung 55 parallel zur Achse 578 bewirkt.

Bevor auf die Funktion der Stangenkupplung 55 mit Bezug auf die Figuren 6 bis 8 näher eingegangen wird, werden anhand der Figuren 4 und 5 zwei verschiedene Ausführungsbeispiele des Schiebers 541 beschrieben. Die Figuren 4 und 5 zeigen jeweils links einen Schnitt entlang der in Figur 3 angedeuteten Schnittebene A-A, in der Mitte und rechts Schnitte entlang der Ebenen B-B und C-C, die in den Figuren 4 und 5 links in den Darstellungen der Schnitte entlang der Ebenen A-A angedeutet sind. Die Schnittebene A-A ist senkrecht zu der in Figur 3 gezeigten Achse 578. Die Schnittebenen B-B und C-C enthalten jeweils die Achse 578. Alle Schnittebenen A-A, B-B und C-C sind senkrecht zueinander.

Beide in den Figuren 4 und 5 dargestellten Ausführungsbeispiele des Schiebers 541 weisen jeweils eine Durchgangsöffnung 543 auf. In einem in den Figuren 3 bis 5 jeweils links dargestellten distalen Abschnitt ist die Oberfläche der Durchgangsöffnung 543 als abschnittsweise konische Anlagefläche 544 für das proximale Ende 32 des Schafts 30 ausgebildet. Die Anlagefläche 544 ist zur Achse 578 rotationssymmetrisch oder im Wesentlichen rotationssymmetrisch und an die Gestalt des proximalen Endes 32 des in Figur 3 gezeigten Schafts 30 angepasst.

Bei dem in Figur 4 dargestellten Ausführungsbeispiel des Schiebers 541 weist die Durchgangsöffnung 543 in einem proximalen Abschnitt zwei ebene und im Wesentlichen rechteckige Gleitflächen 545 auf, die keilförmig von distal nach proximal aufeinander zu laufen. Die Gleitflächen 545 begrenzen an ihren distalen Rändern einen näherungsweise quadratischen Querschnitt der Durchgangsöffnung 543. An ihren proximalen Rändern begrenzen die Gleitflächen 545 einen schmalen rechteckigen Querschnitt, der an den Querschnitt der Klaue 45 des in Figur 3 dargestellten proximalen Endes 42 der Übertragungsstange 40 angepasst ist.

In Figur 4 links im Schnitt entlang der Ebene A-A ist in gestrichelter Linie der Querschnitt der Klaue 45 am proximalen Ende 42 der Übertragungsstange in der in Figur 3 dargestellten Orientierung gezeigt. Diese Orientierung des proximalen Endes 42 und der Klaue 45 entspricht nicht der durch den Querschnitt der Durchgangsöffnung 543 an den proximalen Ränder der Gleitflächen 545 vorgegebenen Orientierung. In Figur 4 in der Mitte und rechts in den Schnitten entlang den Ebenen B-B und C-C ist das proximale Ende 42 der Übertragungsstange mit der Klaue 45 in der Orientierung dargestellt, die durch den Querschnitt der Durchgangsöffnung 543 an ihrem proximalen Ende vorgegeben ist.

Im Schnitt entlang der Ebene B-B ist erkennbar, dass das distale Ende 42 der Übertragungsstange 40, insbesondere die Klaue 45 in Richtung parallel zur Schnittebene B-B und senkrecht zur Achse 578 nicht eng geführt ist. Im Schnitt entlang der Ebene C-C ist erkennbar, dass das distale Ende 42 der Übertragungsstange 40 in Richtung parallel zur Schnittebene C-C und senkrecht zur Achse 578 am Schieber 541 anliegt, wodurch die Orientierung des proximalen Endes 42 der Übertragungsstange 40 vorgegeben ist. In Figur 4 rechts im Schnitt entlang der Ebene C-C ist erkennbar, dass die Übertragungsstange 40 in einem großen, sich an das proximale Ende 42 anschließenden Bereich abgeflacht ist, um ausgehend von der in Figur 4 in der Mitte und rechts dargestellten Position relativ zum Schieber 541 noch weiter nach proximal verschoben werden zu können.

Figur 5 zeigt ein alternatives Ausführungsbeispiel des Schiebers 541, bei dem sich der Querschnitt der Durchgangsöffnung 543 des Schiebers 541 in einem proximalen Bereich von einer Kreisform zu einer rechteckigen Form verändert. Im Gegensatz zum Ausführungsbeispiel der Figur 4 sind vier Gleitflächen 545 vorgesehen, die jeweils nicht rechteckig und nicht eben sind. Die Gleitflächen 545 bewirken ähnlich wie bei dem Ausführungsbeispiel der Figur 4 beim Einführen eines proximalen Endes 42 einer Übertragungsstange 40 von distal nach proximal eine Rotation der Übertragungsstange von einer beliebigen ursprünglichen Orientierung in eine durch den Schieber 541 vorgegebene Orientierung. Um die Darstellung der Gleitflächen 545 auch in den Schnitten B-B und C-C zu ermöglichen, sind in Figur 5 lediglich die Konturen der Übertragungsstange 40 in gestrichelten Linien dargestellt.

Die Figuren 6, 7 und 8 zeigen schematische Darstellungen der Handhabungseinrichtung 50 bei anderen Positionen des Schafts 30 und der Übertragungsstange 40 relativ zur Handhabungseinrichtung 50. Die in den Figuren 6, 7 und 8 dargestellten Schnittebenen entsprechen der Schnittebene der Figur 3. Um die Figuren 6 bis 8 nicht zu überfrachten, sind nur die Bezugszeichen der wichtigsten Merkmale gezeigt.

Bei der in Figur 6 dargestellten Situation sind der Schaft 30 und die Übertragungsstange 40 so weit in die Handhabungseinrichtung 50 eingeschoben, dass das proximale Ende 32 des Schafts 30 am Schieber 541 anliegt. Beim Einschieben des proximalen Endes 32 des Schafts 30 in die Handhabungseinrichtung 50 wurde der Riegel 531 bei Berührung eines konischen Abschnitts des Schafts 30 mit dem Rand der Öffnung 532 im Riegel 531 gegen die Kraft der Feder 533 senkrecht zur Achse 578 in die in Figur 6 gezeigte Position verschoben.

Die Klaue 45 am proximalen Ende 42 der Übertragungsstange 40 liegt bereits zwischen den Greifbacken 551, die sich jedoch noch in einer geöffneten Montagestellung befinden.

Bei den in den Figuren 7 und 8 gezeigten Situationen ist der Schaft 30 vollständig in die Handhabungseinrichtung 50 eingeschoben. Der Riegel 531 greift in die bereits in Figur 6 sichtbare umlaufende Nut 35 an der äußeren Oberfläche des Schafts 30 ein. Durch die Feder 533 wird der Riegel 531 in dieser den Schaft 30 verriegelnden Position gehalten. Die Verriegelung durch den Riegel 531 kann nur durch Druck auf den Entriegelungsknopf 538 gelöst werden. In der in den Figuren 7 und 8 dargestellten verriegelten Position des Schafts 30 ist der Schieber 541 gegen die Kraft der Feder 542 in seine Arbeitsposition 547 geschoben und wird durch den Schaft 30 dort gehalten.

Die in den Figuren 7 und 8 dargestellten Situationen unterscheiden sich dadurch, dass der Stab 561, die Stangenkupplung 55 und die Übertragungsstange 40 sich in unterschiedlichen Positionen befinden. Insbesondere befinden sich bei der in Figur 7 dargestellten Situation die in Figur 1 gezeigten Maulteile 25, 26 in ihren offenen Positionen 252, 262, und das zweite Griffteil 59 befindet sich in der zweiten Arbeitsposition 592. Bei der in Figur 8 dargestellten Situation befinden sich insbesondere die in Figur 1 dargestellten Maulteile 25, 26 in ihren geschlossenen Positionen 251, 261, und das zweite Griffteil 59 befindet sich in seiner ersten Arbeitsposition 591.

In Figur 7 ist erkennbar, dass die Greifbacken 551 an der proximalen Stirnfläche 549 des Schiebers 541 anliegen. Der Schieber 541 bzw. seine proximale Stirnfläche 549 bilden somit einen mechanischen Anschlag für die Greifbacken 551. Die Greifbacken 551 können deshalb gegenüber ihrer in Figur 7 dargestellten Position nicht weiter nach distal verschoben werden. Dadurch bleiben die Greifbacken 551 gesteuert durch die Steuernuten 555, in die die Führungsstifte 554 der Greifbacken 551 eingreifen, (vergleiche Figur 3) in den in den Figuren 7 und 8 gezeigten geschlossenen Arbeitspositionen. In den in den Figuren 7 und 8 dargestellten geschlossenen Arbeitspositionen halten die Greifbacken die Klaue 45 am proximalen Ende 42 der Übertragungsstange 40 formschlüssig und spielarm. Erst nach Entriegelung des Schafts 30 durch Druck auf den Entriegelungsknopf 538 können der Schaft 30, der Schieber 541 und damit auch die Greifbacken 551 so weit nach distal geschoben werden, dass die Greifbacken 551 gesteuert durch die Steuernuten 555 die in Figur 6 dargestellten Montagepositionen erreichen und die Klaue 45 freigeben.

Eine weitere Folge des mechanischen Anschlags der Greifbacken 551 an der proximalen Stirnfläche 549 des Schiebers 541 ist, dass die von den Greifbacken 551 gehaltene Übertragungsstange 40 nicht so weit nach distal verschoben werden kann, dass die Maulteile 25, 26 die in Figur 2 dargestellten überoffenen Positionen 253, 263 erreichen. Damit kann, wie oben anhand der Figur 2 dargestellt, auch das Werkzeug 20 nicht vom distalen Ende 31 des Schafts 30 getrennt werden.

Bei der anhand der Figuren 3 bis 8 dargestellten Ausführungsform weist der Schieber 541 mehrere Funktionen auf. Eine Funktion des Schiebers 541 ist es, während des Einführens des proximalen Endes 42 der Übertragungsstange 40 in die Handhabungseinrichtung 50 das proximale Ende 42 der Übertragungsstange 40 in eine vorbestimmte Orientierung zu rotieren. Eine weitere Funktion des Schiebers 541 besteht darin, die Verschiebbarkeit der Stangenkupplung 55 und der Übertragungsstange 40 nach distal zu begrenzen, wenn der Schaft 30 mittels des Riegels 431 in der Handhabungseinrichtung 50 verriegelt ist. Die Begrenzung der Verschiebbarkeit der Stangenkupplung 55 durch den Schieber 541 bewirkt eine Verriegelung der Stangenkupplung 55 in dem in den Figuren 7 und 8 dargestellten Zustand, in dem die Stangenkupplung 55 das proximale Ende 42 der Übertragungsstange 40 hält. Ferner bewirkt die Begrenzung der Verschiebbarkeit der Stangenkupplung durch den Schieber 541, dass die mit der Stangenkupplung 55 gekoppelte Übertragungsstange 40 nicht so weit nach distal verschoben werden kann, dass die oben anhand der Figuren 1 und 2 beschriebene Verriegelung der mechanischen Kopplung von Werkzeug 20 und Schaft 30 gelöst werden könnte.

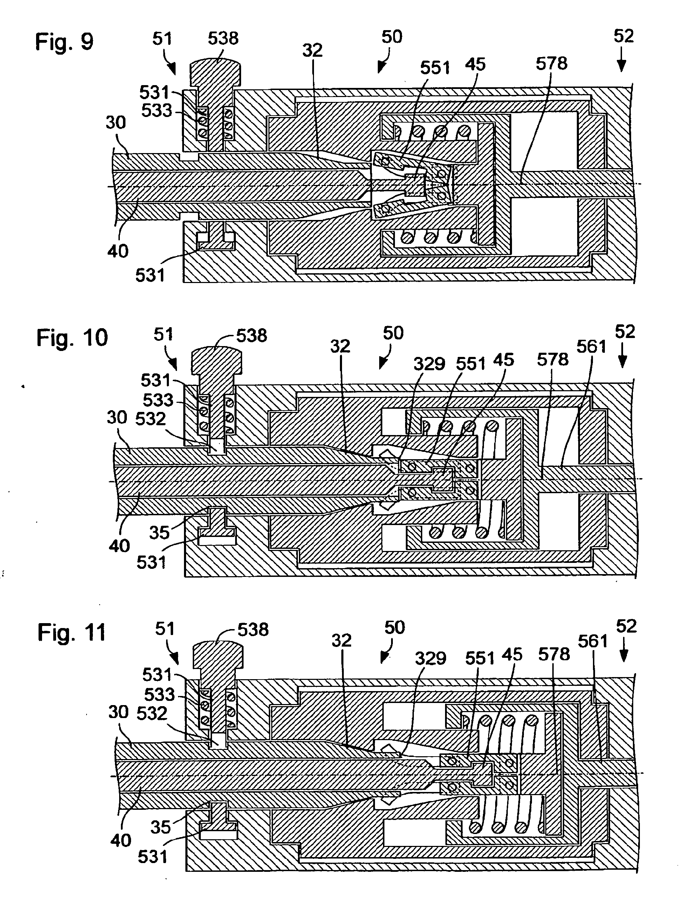

Die Figuren 9 bis 11 zeigen ein alternatives Ausführungsbeispiel der Handhabungseinrichtung 50, das in einigen Merkmalen der oben anhand der Figuren 3 bis 8 dargestellten Handhabungseinrichtung 50 ähnelt. Die Figuren 9 bis 11 zeigen Schnitte entlang Schnittebenen, die den Schnittebenen der Figuren 3 und 6 bis 8 entsprechen. Die in den Figuren 9, 10 und 11 gezeigten Situationen entsprechen den in den Figuren 6, 7 bzw. 8 gezeigten Situationen. Insbesondere zeigt Figur 9 den Schaft 30 noch nicht ganz vollständig in die Handhabungseinrichtung 50 eingeführt und entsprechend auch noch nicht verriegelt. Bei den in den Figuren 10 und 11 gezeigten Situationen ist der Schaft 30 vollständig bis zu einem durch das Drehrad 57 der Handhabungseinrichtung 50 gebildeten Anschlag in die Handhabungseinrichtung 50 eingeführt und durch den Riegel 531 verriegelt. Alternativ bildet abweichend von der Darstellung in den Figuren 7 und 8 das Gehäuse 501 der Handhabungseinrichtung 50 einen mechanischen Anschlag für den Schaft 30.

Die in den Figuren 9 bis 11 gezeigte Handhabungseinrichtung 50 unterscheidet sich von dem oben anhand der Figuren 3 bis 8 dargestellten Ausführungsbeispiel insbesondere dadurch, dass keine Steuer- und Orientierungseinrichtung vorgesehen ist. Stattdessen sind die Handhabungseinrichtung 50 und der Schaft 30, insbesondere das proximale Ende 32 des Schafts 30, so ausgebildet, dass in der in den Figuren 10 und 11 gezeigten verriegelten Position des Schafts 30 eine proximale Stirnfläche 329 am proximalen Ende 32 des Schafts 30 als Anschlag für die Greifbacken 551 wirkt. Ähnlich wie bei dem oben anhand der Figuren 3 bis 8 dargestellten Beispiel sind damit die Greifbacken 551 in den in den Figuren 10 und 11 gezeigten Arbeitspositionen verriegelt, in denen sie die Klaue 45 am proximalen Ende 42 der Übertragungsstange 40 formschlüssig halten.

Ferner kann aufgrund des durch das proximale Ende 32 des Schafts 30 gebildeten Anschlags für die Greifbacken 551 die von den Greifbacken 551 gehaltene Übertragungsstange 40 nicht so weit nach distal verschoben werden, dass die in den Figuren 1 und 2 dargestellten Maulteile 25, 26 die in Figur 2 dargestellten überoffenen Positionen 253, 263 erreichen könnten. Damit ist gleichzeitig die mechanische Verbindung zwischen dem Werkzeug 20 und dem distalen Ende 31 des Schafts 30 verriegelt, wenn der Schaft 30 durch den Riegel 531 in der vorbestimmten Position in der Handhabungseinrichtung 50 gehalten wird. Anders ausgedrückt, verriegelt das proximale Ende 32 des in der Handhabungseinrichtung 50 verriegelten Schafts 30 die Kopplung der Stangenkupplung 55 mit dem proximalen Ende 42 der Übertragungsstange 40 und die mechanische Verbindung zwischen dem Werkzeug 20 und dem distalen Ende 31 des Schafts 30 durch unmittelbare Begrenzung der Verschiebbarkeit der Stangenkupplung 55 auf den Arbeitsbereich, der sich von der in Figur 10 dargestellten Position bis zu der in Figur 11 dargestellten Position erstreckt.

Bei den oben dargestellten Ausführungsformen ist die Übertragungsstange 40 sowohl zur Übertragung einer translatorischen Bewegung in einer Richtung parallel zum Schaft 30 bzw. zu dessen Längsachse und einer entsprechenden Zug- und/oder Schubkraft als auch zur Übertragung einer rotatorischen Bewegung und eines entsprechenden Drehmoments ausgebildet. Eine in Längsrichtung wirkende Kraft wird durch Formschluss zwischen der Stangenkupplung 55 der Handhabungseinrichtung 50 und dem proximalen Ende 42 der Übertragungsstange 40 übertragen. Dazu umfassen insbesondere die Greifbacken 551 der Stangenkupplung 55 die Klaue 45 am proximalen Ende 42 der Übertragungsstange 40 und nehmen diese in ihren Ausnehmungen 553 auf. Eine in Längsrichtung wirkende Kraft kann alternativ oder zusätzlich durch Kraft- bzw. Reibschluss zwischen der Stangenkupplung 55 und dem proximalen Ende 42 der Übertragungsstange übertragen werden. Insbesondere kann eine Übertragung einer in Längsrichtung wirkenden Kraft aufgrund von Kraft- bzw. Reibschluss auch dann erfolgen, wenn das proximale Ende 42 der Übertragungsstange 40 im Bereich der Stangenkupplung 55 anstelle der Klaue 45 einen konstanten Querschnitt aufweist.

Abweichend von der obigen Darstellung anhand der Figuren können die Übertragungsstange 40 und die Stangenkupplung 55 nur zur Übertragung eines Drehmoments ausgebildet sein. Dies kann insbesondere der Fall sein, wenn das Werkzeug 20 am distalen Ende 31 des Schafts 30 lediglich um die Längsachse des distalen Endes 31 des Schafts 30 oder um eine andere Achse rotierbar ist. Beispielsweise umfasst das Werkzeug 20 einen fingerförmigen oder anderen Manipulator oder eine Elektrode in Haken- oder Schlaufenform oder in anderer Gestalt. Insbesondere wenn zwischen Übertragungsstange 40 und Stangenkupplung 55 nur ein Drehmoment übertragen werden soll, kann das proximale Ende 42 der Übertragungsstange 40 abweichend von den Darstellungen in den Figuren 2 bis 8 einen konstanten Querschnitt aufweisen.

### Bezugszeichen

- 10: Mikroinvasiv-chirurgisches Instrument
- 11: distales Ende des mikroinvasiv-chirurgischen Instruments 10
- 12: proximales Ende des mikroinvasiv-chirurgischen Instruments 10
- 19: Richtung des Zusammenbaus
- 20: Werkzeug des mikroinvasiv-chirurgischen Instruments 10
- 21: distales Ende des Werkzeugs 20
- 22: proximales Ende des Werkzeug 20
- 23: Gelenkeinrichtung
- 25: erstes Maulteil des Werkzeugs 20
- 251: geschlossene Position des ersten Maulteils 25
- 252: offene Position des ersten Maulteils 25
- 253: überoffene Position des ersten Maulteils 25
- 26: zweites Maulteil des Werkzeugs 20
- 261: geschlossene Position des zweiten Maulteils 26
- 262: offene Position des zweiten Maulteils 26
- 263: überoffene Position des zweiten Maulteils 26
- 29: Längsachse des Werkzeugs 20
- 30: Schaft des mikroinvasiv-chirurgischen Instruments 10
- 31: distales Ende des Schafts 30
- 32: proximales Ende des Schafts 30
- 329: proximale Stirnfläche des Schafts 30
- 34: Kanal im Schaft 30
- 35: umlaufende Nut nahe dem proximalen Ende 32 des Schafts 30

- 40: Übertragungsstange des mikroinvasiv-chirurgischen Instruments 10
- 41: distales Ende der Übertragungsstange 40
- 42: proximales Ende der Übertragungsstange 40
- 45: Klaue am proximalen Ende 42 der Übertragungsstange 40

- 50: Handhabungseinrichtung des mikroinvasiv-chirurgischen Instruments 10
- 501: Gehäuse der Handhabungseinrichtung 50
- 503: Ausnehmung in Gehäuse 501
- 51: distales Ende der Handhabungseinrichtung 50
- 52: proximales Ende der Handhabungseinrichtung 50

- 53: Schaftkupplung der Handhabungseinrichtung 50
- 530: Führungsfläche der Schaftkupplung 530
531 Riegel der Schaftkupplung 53
532 Öffnung im Riegel 530
533 Feder am Riegel 530
534 Kragen am Riegel 530
538 Entriegelungsknopf am Riegel 530
54 Steuer- und Orientierungseinrichtung
541 Schieber
542 Feder am Schieber 541
543 Durchgangsöffnung am Schieber 541
544 Anlagefläche für proximales Ende 32 des Schafts 30
545 Gleitfläche am Schieber 541
546 distale Montageposition des Schiebers 541
547 proximale Arbeitsposition des Schiebers 541
549 proximale Stirnfläche des Schiebers 541
55 Stangenkupplung der Handhabungseinrichtung 50
551 Greifbacke der Übertragungsstangenkupplung 55
552 Gelenk an der Greifbacke 551
553 Ausnehmung an der Greifbacke 551
554 Führungsstift an der Greifbacke 551
555 Steuernut für den Führungsstift 554
561 Stab
562 becherförmiges Bauteil
563 Kragen am becherförmigen Bauteil 562
564 Druckfeder
565 Kraftübertragungsbauteil
57 Drehrad
578 Achse
58 erstes Griffteil der Handhabungseinrichtung 50
59 zweites Griffteil der Handhabungseinrichtung 50
591 erste Arbeitsposition des zweiten Griffteils 59
592 zweite Arbeitsposition des zweiten Griffteils 59

## Patentansprüche

1. **Handhabungseinrichtung** (50) für ein mikroinvasiv-chirurgisches Instrument (10), mit:
einer **Griffeinrichtung** (58, 59) zum manuellen Halten und Führen der Handhabungseinrichtung (50);
einer **Schaftkupplung** (53) zum lösbaren mechanischen Koppeln der Handhabungseinrichtung (50) mit einem proximalen Ende (32) eines Schafts (30);
einer **Stangenkupplung** (55), die in der **Handhabungseinrichtung** zwischen einer vorbestimmten distalen Montageposition und einem vorbestimmten proximalen Bereich von Arbeitspositionen linear verschiebbar ist, zum lösbaren mechanischen Koppeln mit einem proximalen Ende (42) einer Übertragungsstange (40), die in einem mit der Schaftkupplung (53) gekoppelten Schaft (50) zumindest entweder verschiebbar oder rotierbar ist,
wobei die verschiebbare Stangenkupplung (55) ausgebildet ist, um in der vorbestimmten **Montageposition** ein proximales Ende (42) einer Übertragungsstange (40) aufzunehmen oder freizugeben, und um in dem vorbestimmten **Arbeitsbereich** von Positionen der Stangenkupplung (55) ein in die Stangenkupplung (55) eingesetztes proximales Ende einer Übertragungsstange (40) zu halten,
wobei die Schaftkupplung (53) ausgebildet ist, um ein in die Schaftkupplung (53) eingesetztes proximales Ende (32) eines Schafts (30) in einer vorbestimmten Position zu arretieren, **dadurch gekennzeichnet, dass** das proximale Ende (32) des Schafts (30) die Stangenkupplung (540) **in dem vorbestimmten Bereich von Arbeitspositionen** hält.

2. Handhabungseinrichtung (50) nach dem vorangehenden Anspruch, ferner mit:
einem Schieber (541), der in der Handhabungseinrichtung (50) zwischen einer vorbestimmten distalen Montageposition (546) und einer vorbestimmten proximalen Arbeitsposition (547) **verschiebbar** ist, wobei der Schieber (541) ausgebildet und angeordnet ist, um von einem in der Schaftkupplung (53) arretierten proximalen Ende (32) eines Schafts (30) in der vorbestimmten proximalen Arbeitsposition (547) gehalten zu werden.

3. Handhabungseinrichtung (50) nach dem vorangehenden Anspruch, bei der der Schieber (541) eine **Durchgangsöffnung** (543) aufweist, die sich von proximal nach distal erstreckt, wobei der Querschnitt der Durchgangsöffnung (543) von distal nach proximal zumindest abschnittsweise stetig variiert.

4. Handhabungseinrichtung (50) nach Anspruch 2 oder 3, bei der
der Schieber (541) relativ zu der Handhabungseinrichtung (50) um eine Achse (578) **rotierbar** ist,
die Schieber (541) ausgebildet ist, um ein **Drehmoment** auf eine mit der Stangenkupplung (55) gekoppelte Übertragungsstange (40) zu übertragen.

5. Handhabungseinrichtung (50) nach einem der vorangehenden Ansprüche, bei der die Stangenkupplung (55) eine bewegbare **Greifbacke** (551) zum Halten eines proximalen Endes (42) einer Übertragungsstange (40) umfasst, wobei die Stangenkupplung (55) so ausgebildet ist, dass die Greifbacke (551) bei einer vorbestimmten Montageposition der Stangenkupplung (55) ein proximales Ende (42, 45) einer Übertragungsstange (40) aufnehmen oder freigeben und in einem vorbestimmten Arbeitsbereich von Positionen der Stangenkupplung (55) ein proximales Ende (42) einer Übertragungsstange (40) halten kann.

6. Handhabungseinrichtung (50) nach dem vorangehenden Anspruch, ferner mit:
einem Führungsstift (554) und einer Steuernut (555), wobei entweder der Führungsstift (554) oder die Steuernut (555) an der Greifbacke (551) angeordnet ist, und wobei der Führungsstift (554) in die Steuernut (555) eingreift, um abhängig von der Position der Stangenkupplung (55) die Greifbacke (551) in unterschiedlichen Positionen zu halten.

7. Handhabungseinrichtung (50) nach einem der Ansprüche 5 und 6 in Rückbezug auf einen der Ansprüche 2 bis 4, bei der ein Oberflächenbereich (549) am Schieber (541) ausgebildet ist, um in der vorbestimmten proximalen Arbeitsposition (547) des Schiebers (541) einen mechanischen Anschlag für die Greifbacke (551) der Stangenkupplung (55) zu bilden, der den vorbestimmten Arbeitsbereich begrenzt.

8. **Mikroinvasiv-chirurgisches Instrument**(10), mit:
einer **Handhabungseinrichtung** (50) nach einem der vorangehenden Ansprüche;
einem **Schaft** (30) mit einem proximalen Ende (32), das zur lösbaren Kopplung mit der Handhabungseinrichtung (50) ausgebildet ist, und einem distalen Ende (31), das mit einem Werkzeug (20) mechanisch verbunden oder verbindbar ist;
einer **Übertragungsstange** (40), zum Übertragen zumindest entweder einer Kraft oder eines Drehmoments von der Handhabungseinrichtung (50) zum distalen Ende (31) des Schafts (30).

9. Mikroinvasiv-chirurgisches Instrument nach dem vorangehenden Anspruch, bei dem die Übertragungsstange (40) ferner ausgebildet ist, um ein **Drehmoment** von der Handhabungseinrichtung (50) zum distalen Ende (31) des Schafts (30) zu **übertragen.**

10. Mikroinvasiv-chirurgisches Instrument nach Anspruch 8 oder 9, bei dem das Werkzeug (20) und das distale Ende (31) des Schafts (30) lösbar miteinander mechanisch koppelbar sind, wobei Werkzeug (20), Schaft (30), und Übertragungsstange (40) so ausgebildet sind, dass das Werkzeug (20) am distalen Ende (31) des Schafts (30) verriegelt ist, wenn die Position der mit dem proximalen Ende (42) der Übertragungsstange (40) gekoppelte Stangenkupplung (55) in dem vorbestimmten Arbeitsbereich liegt.

## Claims

1. Handling device (50) for a micro-invasive surgical instrument (10), with:
a grip device (58, 59) for manual holding and guiding of the handling device (50);
a shank coupling (53) for detachable mechanical coupling of the handling device (50) to a proximal end (32) of a shank (30);
a rod coupling (55) which is linearly displaceable in the handling device between a predetermined distal mounting position and a predetermined proximal area of working positions, for detachable mechanical coupling to a proximal end (42) of a transmission rod (40), which is at least either displaceable or rotatable in a shank (50) coupled to the shank coupling (53),
wherein the displaceable rod coupling (55) is designed to receive or release, in the predetermined mounting position, a proximal end (42) of a transmission rod (40) and, in the predetermined working area of positions of the rod coupling (55), is designed to hold a proximal end of a transmission rod (40) inserted into the rod coupling (55),
wherein the shank coupling (53) is designed to lock a proximal end (32) of a shank (30) in a predetermined position when the proximal end (32) is inserted into the shank coupling (53), **characterized in that** the proximal end (32) of the shank (30) holds the rod coupling (55) in the predetermined area of working positions.

2. Handling device (50) according to the preceding claim, also with:
a slider (541), which is displaceable in the handling device (50) between a predetermined distal mounting position (546) and a predetermined proximal working position (547), wherein the slider (541) is designed and arranged to be held in the predetermined proximal working position (547) by a proximal end (32) of a shank (30) locked in the shank coupling (53).

3. Handling device (50) according to the preceding claim, in which the slider (541) has a through-opening (543), which extends from proximal to distal, wherein the cross section of the through-opening (543) constantly varies, at least in some parts, from distal to proximal.

4. Handling device (50) according to Claim 2 or 3, in which
the slider (541) is rotatable about an axis (578) relative to the handling device (50),
the slider (541) is designed to transmit a torque to a transmission rod (40) coupled to the rod coupling (55).

5. Handling device (50) according to one of the preceding claims, in which the rod coupling (55) comprises a movable clamping jaw (551) for holding a proximal end (42) of a transmission rod (40), wherein the rod coupling (55) is designed such that the clamping jaw (551), in a predetermined mounting position of the rod coupling (55), can receive or release a proximal end (42, 45) of a transmission rod (40) and, in a predetermined working area of positions of the rod coupling (55), can hold a proximal end (42) of a transmission rod (40).

6. Handling device (50) according to the preceding claim, also with:
a guide pin (554) and a control groove (555), wherein either the guide pin (554) or the control groove (555) is arranged on the clamping jaw (551), and wherein the guide pin (554) engages in the control groove (555) in order to hold the clamping jaw (551) in different positions depending on the position of the rod coupling (55).

7. Handling device (50) according to either of Claims 5 and 6 in back-reference to one of Claims 2 to 4, in which a surface area (549) on the slider (541) is designed to form, in the predetermined proximal working position (547) of the slider (541), a mechanical stop for the the clamping jaw (551) of the rod coupling (55), which mechanical stop limits the predetermined working area.

8. Micro-invasive surgical instrument (10), with:
a handling device (50) according to one of the preceding claims;
a shank (30) with a proximal end (32), which is designed for detachable coupling to the handling device (50), and with a distal end (31), which is mechanically connected or connectable to a tool (20);
a transmission rod (40) for transmitting at least either a force or a torque from the handling device (50) to the distal end (31) of the shank (30).

9. Micro-invasive surgical instrument according to the preceding claim, in which the transmission rod (40) is further designed to transmit a torque from the handling device (50) to the distal end (31) of the shank (30).

10. Micro-invasive surgical instrument according to Claim 8 or 9, in which the tool (20) and the distal end (31) of the shank (30) can be detachably coupled to each other mechanically, wherein tool (20), shank (30) and transmission rod (40) are designed such that the tool (20) is locked on the distal end (31) of the shank (30) when the position of the rod coupling (55) coupled to the proximal end (42) of the transmission rod (40) lies in the predetermined working area.

## Revendications

1. Dispositif de manipulation (50) pour un instrument de chirurgie micro-invasive (10), comprenant :
un dispositif de préhension (58, 59) pour retenir et guider manuellement le dispositif de manipulation (50) ;
un accouplement d'arbre (53) pour l'accouplement mécanique détachable du dispositif de manipulation (50) à une extrémité proximale (32) d'un arbre (30) ;
un accouplement de tige (55), qui peut être déplacé linéairement dans le dispositif de manipulation entre une position de montage distale prédéterminée et une région proximale prédéterminée de positions de travail, pour l'accouplement mécanique détachable à une extrémité proximale (42) d'une tige de transfert (40) qui peut être au moins soit déplacée soit tournée dans un arbre (50) accouplé à l'accouplement d'arbre (53),
l'accouplement de tige déplaçable (55) étant réalisé pour recevoir ou libérer, dans la position de montage prédéterminée, une extrémité proximale (42) d'une tige de transfert (40), et pour retenir, dans la région de travail prédéterminée de positions de l'accouplement de tige (55), une extrémité proximale d'une tige de transfert (40) insérée dans l'accouplement de tige (55),
l'accouplement d'arbre (53) étant réalisé pour bloquer une extrémité proximale (32) d'une tige (30) insérée dans l'accouplement d'arbre (53) dans une position prédéterminée, **caractérisé en ce que** l'extrémité proximale (32) de l'arbre (30) retient l'accouplement de tige (55) dans la région prédéterminée de positions de travail.

2. Dispositif de manipulation (50) selon la revendication précédente, comprenant en outre :
un coulisseau (541) qui peut coulisser dans le dispositif de manipulation (50) entre une position de montage distale prédéterminée (546) et une position de travail proximale prédéterminée (547), le coulisseau (541) étant réalisé et disposé de manière à être retenu par une extrémité proximale (32) d'une tige (30) bloquée dans l'accouplement d'arbre (53) dans la position de travail proximale prédéterminée (547).

3. Dispositif de manipulation (50) selon la revendication précédente, dans lequel le coulisseau (541) présente une ouverture de passage (543) qui s'étend de la position proximale vers la position distale, la section transversale de l'ouverture de passage (543) variant constamment au moins en partie de la position distale vers la position proximale.

4. Dispositif de manipulation (50) selon la revendication 2 ou 3, dans lequel
le coulisseau (541) peut tourner autour d'un axe (578) par rapport au dispositif de manipulation (50), et
le coulisseau (541) est réalisé pour transférer un couple à une tige de transfert (40) accouplée à l'accouplement de tige (55).

5. Dispositif de manipulation (50) selon l'une quelconque des revendications précédentes, dans lequel l'accouplement de tige (55) comprend une mâchoire de préhension mobile (551) pour retenir une extrémité proximale (42) d'une tige de transfert (40), l'accouplement de tige (55) étant réalisé de telle sorte que la mâchoire de préhension (551), dans une position de montage prédéterminée de l'accouplement de tige (55), puisse recevoir ou libérer une extrémité proximale (42, 45) d'une tige de transfert (40) et, dans une région de travail prédéterminée de positions de l'accouplement de tige (55), puisse retenir une extrémité proximale (42) d'une tige de transfert (40).

6. Dispositif de manipulation (50) selon la revendication précédente, comprenant en outre :
une goupille de guidage (554) et une rainure de commande (555), soit la goupille de guidage (554) soit la rainure de commande (555) étant disposée sur la mâchoire de préhension (551), et la goupille de guidage (554) s'engageant dans la rainure de commande (555) afin de retenir, en fonction de la position de l'accouplement de tige (55), la mâchoire de préhension (551) dans différentes positions.

7. Dispositif de manipulation (50) selon l'une quelconque des revendications 5 et 6, lorsqu'elles se rapportent à l'une quelconque des revendications 2 à 4, dans lequel une région de surface (549) est réalisée sur le coulisseau (541) afin de former, dans la position de travail proximale prédéterminée (547) du coulisseau (541), une butée mécanique pour la mâchoire de préhension (551) de l'accouplement de tige (55), laquelle limite la région de travail prédéterminée.

8. Instrument de chirurgie micro-invasive (10), comprenant :
un dispositif de manipulation (50) selon l'une quelconque des revendications précédentes ;
un arbre (30) avec une extrémité proximale (32) qui est réalisée pour l'accouplement détachable au dispositif de manipulation (50), et une extrémité distale (31) qui est connectée ou peut être connectée mécaniquement à un outil (20) ;
une tige de transfert (40) pour transférer au moins soit une force soit un couple du dispositif de manipulation (50) à l'extrémité distale (31) de l'arbre (30).

9. Instrument de chirurgie micro-invasive selon la revendication précédente, dans lequel la tige de transfert (40) est en outre réalisée pour transférer un couple du dispositif de manipulation (50) à l'extrémité distale (31) de l'arbre (30).

10. Instrument de chirurgie micro-invasive selon la revendication 8 ou 9, dans lequel l'outil (20) et l'extrémité distale (31) de l'arbre (30) peuvent être accouplés mécaniquement de manière détachable l'un à l'autre, l'outil (20), l'arbre (30) et la tige de transfert (40) étant réalisés de telle sorte que l'outil (20) soit verrouillé à l'extrémité distale (31) de l'arbre (30) lorsque la position de l'accouplement de tige (55) accouplé à l'extrémité proximale (42) de la tige de transfert (40) est située dans la région de travail prédéterminée.
